# FASCICULE DE BREVET EUROPEEN

(11) **EP 1 276 735 B1**
(45) Date de publication et mention de la délivrance du brevet: **08.06.2005**
(21) Numéro de dépôt: 01914850.1
(22) Date de dépôt: 22.03.2001
(51) Int. Cl.: C07D 319/12

(54) **PROCEDE DE PURIFICACTION D'ESTERS CYCLIQUES**
VERFAHREN ZUR REINIGUNG VON ZYKLISCHEN ESTERN
METHOD FOR PURIFYING CYCLIC ESTERS

(30) Priorité: 23.03.2000 EP 00870052
(43) Date de publication de la demande: 22.01.2003
(73) Titulaire: Brussels Biotech, 1180 Bruxelles (BE)
(72) Inventeur: VAN GANSBERGHE, Frédéric, 1180 Bruxelles (BE); DI SALVATORE, Patricia, 1180 Bruxelles (BE); BOGAERT, Jean-Christophe, 1180 Bruxelles (BE); COSZACH, Philippe, 1180 Bruxelles (BE)
(74) Mandataire: Van Straaten, Joop
(86) Numéro de dépôt international: PCT/BE2001/000047
(87) Numéro de publication internationale: WO 2001/070721

(56) Documents cités:
- EP-A- 0 657 447
- FR-A- 2 701 707

## Description

### Domaine de l'invention

La présente invention concerne un procédé de purification d'esters cycliques dimériques (en particulier lactides ou glycolides) de formule générale : 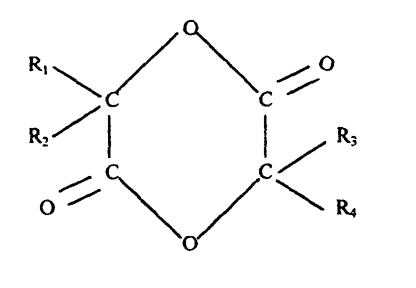 où R₁, R₂, R₃ et R₄ peuvent être soit un hydrogène, soit un groupement aromatique, soit un groupement aliphatique substitué ou non, ayant de 1 à 10 atomes de carbone. Ces esters sont transformables en polymères particulièrement utiles pour la préparation de plastiques biodégradables et résorbables en médecine. Les polymères de lactide (ou R₁ = R₃ = H et R₂ = R₄ = CH₃) sont dégradables par hydrolyse aqueuse dans la plupart des conditions environnementales, en acide lactique ou en oligomères.

Les deux formes optiquement actives d'acide lactique (L-LA) et (D-LA) peuvent donner un lactide (LD ou dimère cyclique) sous 3 formes diastéréoisomériques : avec 2 molécules d'acide D-lactique (D,D-lactide ou D-LD), avec 2 molécules d'acide L-lactique (L,L-lactide ou L-LD), ou avec une molécule de chaque (méso-lactide ou méso-LD). On rencontre aussi le mélange racémique ((D,L)-lactide) caractérisé par une température de fusion (Tf = 126°C) supérieure à celle du L-LD ou D-LD (Tf = 97°C).

Actuellement, les 2 grandes méthodes de production de lactide se distinguent essentiellement par le degré de polymérisation moyen (DP) des oligomères de l'étape de condensation.

La première consiste à extraire l'eau d'une solution d'acide lactique jusqu'à obtenir des oligomères avec 8 ≤ DP ≤ 25. Ensuite, on dépolymérise ces oligomères (réaction de back-bitting) avec un catalyseur acide de Lewis, soit sous pression réduite à température plus ou moins élevée, soit sous flux d'azote. Ce procédé se réalise dans des conditions drastiques qui s'avèrent coûteuses et influent sur la pureté optique du lactide (pourcentage élevé de racémisation).

La seconde méthode utilise un oligomère avec 1,5 ≤ DP ≤ 2,5 produit en phase vapeur à température élevée ou en phase liquide en présence d'un co-solvant formant un azéotrope avec l'eau. Les principaux inconvénients sont la présence d'un solvant souvent aromatique et de haut point d'ébullition, une température de réaction > 180°C, un manque de sélectivité et une quantité non négligeable d'impuretés protiques.

De manière générale, le brut de lactide obtenu par diverses voies de synthèse contient une série d'impuretés protiques (acides carboxyliques, composés hydroxylés, eau, etc.) qu'il faudra extraire afin d'obtenir une pureté suffisante pour intégrer le brut au procédé de polymérisation par ouverture de cycle.

### Etat de la technique

Les pourcentages seront toujours exprimés en poids dans la suite de ce texte.

L'homme de l'art connaît CA 2 115 472 qui propose un procédé de purification par cristallisation dynamique à l'état fondu, avec récupération du méso-LD sous une forme enrichie. Mais le procédé n'est applicable qu'à des bruts de lactide présentant des rapports L-LD/D-LD d'au moins 80/20 ou d'au moins 20/80. Si la composition du brut se trouve correspondre à une composition au-delà de l'eutectique, on assiste alors à un enrichissement en mélange racémique L-LD + D-LD, avec rejet du L-LD avec les impuretés comme l'eau, l'acide lactique et les oligomères. D'autre part, il est nécessaire de partir d'un brut de lactide déjà riche en lactides (>90% L-LD + méso-LD).

US 5,502,215 (EP 0 657 447) concerne un procédé de purification de brut de lactide, comprenant une cristallisation en milieu aqueux de L-LD et/ou D-LD, puis une séparation centrifuge, un séchage en phase gazeuse et une recristallisation en solvant organique avec séparation centrifuge et séchage en phase gazeuse et éventuellement un rinçage avec ce solvant organique. L'accent est mis sur l'élimination par hydrolyse du méso-LD et non sur une extraction des impuretés protiques par l'eau. D'autre part, on ne cherche pas à produire de cristaux de lactide d'un type particulier.

Les deux méthodes de purifications décrites ci-dessus, permettent de traiter divers bruts de lactide et d'obtenir des puretés de l'ordre de 99 %, autorisant une polymérisation en polylactide (PLA) dans des conditions raisonnables. Mais, ces méthodes entraînent soit des pertes de rendement importantes dues à l'ouverture, la racémisation chimique et/ou thermique du cycle lactide, soit des investissements et des coûts d'exploitation importants suite aux nécessités de stockage et de traitement liées à une purification avec solvant.

La présente invention pallie ces inconvénients, tout en permettant de produire un lactide suffisamment pur en vue de la polymérisation dans de bonnes conditions économiques.

### Brève description de l'invention

La présente invention consiste en un procédé de purification d'esters cycliques, en particulier du dimère cyclique de l'acide lactique (le lactide) au départ d'un "brut de lactide" à savoir un mélange d'acide lactique et/ou d'ester d'acide lactique et de leurs oligomères respectifs (LₙA avec n < 5), d'eau et/ou d'alcool ainsi que des différentes formes diastéréoisomériques du lactide.

Ce brut peut être obtenu soit au départ d'acide lactique, et/ou de ses sels et/ou de ses esters provenant de toute synthèse connue de l'homme de l'art et dont une description non exhaustive a été développée ci-avant, soit au départ de résidus de procédés de purification comme la distillation ou la cristallisation en milieu fondu.

Dans la suite nous ferons toujours référence à la synthèse de lactide au départ d'acide lactique, mais elle pourra également s'appliquer aux esters d'acide lactique. Par lactide on entend l'une des deux formes diastéréoisomériques (le L-LD ou le D-LD) et non le méso-LD.

Le procédé de purification décrit dans cette invention est original car il donne, au départ d'un brut de lactide (même peu riche en lactide), une très haute qualité de lactide avec un rendement massique élevé et une consommation énergétique minimale. Un lactide de très haute qualité (chimique ou optique) peut servir comme monomère pour la synthèse du PLA par ouverture de cycle.

Le procédé quantitatif et sélectif est assuré par la mise en oeuvre conjointe : (a) d'une cristallisation extractive et contrôlée du lactide en milieu aqueux afin de favoriser la formation de gros cristaux ainsi que le transfert des impuretés protiques en phase liquide, (b) d'une séparation centrifuge ou autre (avec ou sans lavage) du lactide et de la phase aqueuse, (c) d'un séchage en phase solide ou liquide du gâteau humide obtenu et (d) d'une ou plusieurs recristallisations en milieu fondu.

Cette séquence permet un recyclage aisé et quantitatif des impuretés en phase aqueuse au sein de la production d'acide lactique. L'optimisation des conditions de température et de temps de séjour permet, contrairement aux procédés conventionnels, d'éviter les détériorations chimiques et thermiques du lactide durant la purification. Les critères de qualité et de rendement industriels sont atteints beaucoup plus facilement.

La dépense d'énergie est minimale grâce à la simplicité des technologies, aux faibles températures de travail et à la judicieuse juxtaposition des étapes. La recristallisation en milieu fondu est connue de l'homme de l'art, car elle permet d'obtenir un lactide d'une qualité irréprochable et une sélectivité requise pour la synthèse du PLA. Cependant, au départ d'un brut de lactide pauvre en lactide, cette technologie ne peut à la fois garantir un rendement suffisant en lactide et soutenir une comparaison économique vis-à-vis d'autres technologies (distillation, recristallisation en solvant, etc.). Par contre, la succession des étapes (a) à (d) et la méthodologie préconisées par la présente invention compensent le handicap.

### Description détaillée de l'invention

De manière préférée, le mélange de départ aura une composition en un des lactides comprise entre 30 et 90 % et préférablement entre 40 et 85 %, en eau (lors d'un travail avec un ester, l'eau sera remplacée par un alcool) entre 0 et 2 % et préférablement entre 0 et 1 %, en acide lactique et ses oligomères (LₙA avec n < 5) entre 0 et 50 %, le solde (le méso-LD et l'autre diastéréoisomère du lactide) entre 0 et 30 %.

Ce mélange ou brut provient de l'extraction à un moment précis de l'étape de condensation des vapeurs issues de la synthèse du dimère cyclique de l'acide lactique. On peut aussi récupérer les fractions issues de procédés de purification (distillation ou cristallisation en milieu fondu) dont la teneur en lactide est trop faible pour être purifiées mais suffisante pour un recyclage comme lactide et non comme source d'acide lactique (hydrolyse du lactide).

Ce procédé comprend essentiellement les étapes suivantes :

### (a) une cristallisation extractive et contrôlée

Elle consiste en une cristallisation, quantitative, sélective et contrôlée en milieu aqueux du lactide avec concentration des impuretés protiques en phase liquide, par ajout d'eau.

Couplée à celle de la séparation centrifuge (b) et celle de séchage (c), elle constitue une prépurification donnant un mélange de sélectivité (teneur en LD) suffisante pour une purification ultime, efficace et rentable, par la recristallisation en milieu fondu (d). La sélectivité élevée donnera une teneur en LD supérieure à 90 % et préférablement supérieure à 95 %, sans tenir compte de l'eau ajoutée.

Par rapport aux procédés d'extraction à l'eau déjà connus, on n'élimine pas ici la majeure partie du méso-LD par hydrolyse du cycle, mais on contrôle la géométrie des cristaux formés, on provoque une ségrégation de phases entre le lactide (phase solide) et les impuretés (phase liquide) et on favorise l'extraction des impuretés protiques solubles. Une diminution de la teneur en méso-LD ne pourra pas être totalement évitée. L'étape ultime du procédé permettra une séparation effective et stéréospécifique du lactide et du méso-LD; il faut donc éviter d'hydrolyser ce dernier par ouverture de cycle. Pour certaines applications, la récupération du méso-LD et son utilisation en PLA constituent un atout majeur.

Ce procédé permet un recyclage des impuretés vers la production de lactide à partir d'acide lactique, un contrôle de la géométrie des cristaux, une extraction aqueuse efficace des impuretés protiques, et des conditions réactionnelles très douces pour éviter les pertes de rendement par ouverture chimique ou thermique du lactide.

La présente invention préconise des températures initiales et finales du mélange [brut de lactide + eau servant à l'extraction] n'excédant pas respectivement 100°C et 50°C, préférablement inférieures à 90°C et 35°C et plus préférablement encore inférieures à 80°C et 25°C, et des temps de séjour compris entre 1 et 90 min, de préférence compris entre 1 et 60 min pour initier et compléter l'extraction, afin de réduire la racémisation ainsi que les dépenses énergétiques et augmenter la productivité du procédé.

Mais l'ajout d'une quantité inadaptée d'eau crée des problèmes de transfert, d'efficacité de l'extraction ou de contrôle de la cristallisation. En effet, une quantité trop importante d'eau permet une hydrolyse du méso-LD mais également une hydrolyse du lactide par ouverture de cycle (ce qui influence considérablement le rendement). Dans les procédés d'extraction connus, cette dégradation est freinée par une diminution très rapide de la température du mélange, qui provoque une nucléation très importante au sein du mélange et une prise en masse. Ceci ne gêne pas les procédés d'extraction connus qui visent une hydrolyse sélective du méso-LD présent sous forme cristalline et non une extraction aqueuse des impuretés protiques (la pureté du produit de départ étant déjà relativement élevée). Par contre, en ce qui concerne l'invention, la nucléation et la croissance des cristaux doivent être contrôlées pour éviter la prise en masse qui réduit l'efficacité de l'extraction : la chute de température provoque une hausse de la viscosité des impuretés (tels l'acide lactique ou les oligomères d'acide lactique) qui s'éliminent beaucoup plus difficilement de la surface des cristaux et aussi, la formation d'un bloc difficile à solvater par l'eau.

De plus, la prise en masse empêche le contrôle de la géométrie des cristaux, qui ne pourront développer une structure lamellaire. Ces cristaux suivant l'invention, sans inclusions ni occlusions, sont plus purs, stables et manipulables.

Pour ne pas favoriser une hydrolyse du méso-LD, dans cette invention, la concentration en eau ajoutée au mélange de départ sera moindre, entre 0 et 40 %, préférablement entre 0 et 30 % et plus préférablement entre 0 et 20 %. La dégradation du lactide est alors beaucoup plus lente et permet un meilleur contrôle de la température et de la cristallisation, car à chaque composition en lactide du brut de lactide correspond une température de cristallisation spécifique. Dans une première phase, le mélange sera amené à 10°C, préférablement à 5°C et préférablement encore à 2°C en dessous de cette température et y sera maintenu entre 1 et 45 min, préférablement entre 1 et 30 min, et plus préférablement encore entre 1 et 15 min. Une approche de l'invention consiste à fixer la température de l'eau ajoutée de sorte qu'une fois le mélange effectué, sa température corresponde à la température de maintien souhaitée. Une seconde approche de l'invention consiste à initier la cristallisation des cristaux de lactide pur (germination progressive).

Durant la phase suivante de la première étape, la température du mélange est lentement diminuée pour engendrer la croissance progressive des cristaux et augmenter le rendement. Ce contrôle de la cristallisation repousse progressivement les impuretés en phase liquide et des cristaux à structure lamellaire sans inclusions sont formés. La géométrie de cristaux obtenus augmente sensiblement l'efficacité des deux étapes de séparation (b) et de séchage (c). D'autre part, avec une teneur en eau voisine de 1 %, la stabilité chimique des cristaux de lactide est accrue vis-à-vis de ceux obtenus par prise en masse du mélange.

Dans la présente invention, le réacteur assurera une bonne agitation pour répartir la chaleur sur l'ensemble du mélange, évitera la prise en masse et permettra l'évacuation facile du mélange hors du réacteur. Sa capacité de thermostatisation importante favorisera une cristallisation progressive (rendement) et contrôlée (croissance des cristaux) du lactide. Tout réacteur par charge ou en continu, conforme à ces exigences convient, comme un réacteur par charge couplé à un échangeur externe de chaleur. De plus, l'homme de l'art peut également envisager la sonocristallisation ou l'ensemencement par cristaux, pour favoriser la cristallisation du lactide.

Contrairement aux procédés d'extraction à l'eau connus, cette nouvelle méthode de travail favorise l'apparition d'une forme particulière de lactide.

L'analyse par GC des produits issus de cette cristallisation extractive et contrôlée, à permis d'observer l'apparition d'un composé supplémentaire et inconnu.

En fonction des températures, de la concentration en eau et du temps de contact, la teneur de ce composé évoluait de façon inverse à celle du lactide.

Des analyses par GC-MS, RMN (¹³C, ¹H) et IR, ont montré que ce composé était une molécule de lactide « complexée » par une molécule d'eau. Ce complexe suppose une interaction polaire relativement forte mais pas un lien chimique covalent, car l'analyse des deux molécules (le lactide et le complexe) a fourni deux spectres de masse totalement identiques, sans pic supplémentaire à m/z 162, ce qui tend à prouver l'existence d'un complexe et non d'un lien chimique. Les spectres RMN et IR montrent une modification correspondant bien à la présence d'eau.

Ce complexe n'est pas généré en cas de refroidissement brusque. Or seule la connaissance de l'existence et de la nature de ce complexe permet une gestion correcte et efficace des étapes de séchage et de cristallisation en milieu fondu liées à ce procédé.

### (b) une séparation centrifuge

Cette étape consiste au départ d'une suspension obtenue en (a) et dont la teneur en eau est comprise entre 1 et 40 % en poids, préférablement entre 1 et 25 % et plus préférablement encore entre 1 et 20 %, la teneur en un lactide (complexe inclus) comprise entre 35 et 90 %, préférablement entre 40 et 90 % et plus préférablement encore entre 45 et 90 %, la teneur en acide lactique et ses oligomères (LₙA avec n<5) comprise entre 0 et 10 % et préférablement entre 0 et 5 %, le solde étant du Méso-LD et l'autre diastéréoisomère du lactide, en une séparation centrifuge ou autre du lactide, essentiellement présent dans la phase solide (gâteau), et des filtrats aqueux chargés en impuretés protiques.

Les filtrats pourront être facilement recyclés vers la production de lactide à partir d'acide lactique, pour augmenter le rendement global de synthèse du PLA.

Une séparation centrifuge est souhaitable : elle très rapide en raison de la géométrie favorable des cristaux générés dans l'étape (a). De plus, la siccité du gâteau permet une manutention aisée du produit. D'autre part la stabilité chimique des cristaux évite les pertes de rendement par ouverture de cycle.

Une approche privilégiée recommande un temps d'essorage suffisant pour atteindre des teneurs en eau résiduelle libre comprises entre 0 et 3%, préférablement entre 0 et 1% et plus préférablement entre 0 et 0,5%.

Afin de faciliter ou de supprimer l'étape suivante de séchage, on procède au lavage du gâteau d'essorage. Le lavage permet de réduire les temps de contact au minimum et augmente le rendement. Tout procédé conviendra dans le cadre de cette invention.

Le choix du solvant de lavage permet d'éliminer simplement les impuretés déposées sous forme de film à la surface des cristaux, de réduire la teneur en eau résiduelle du gâteau et d'augmenter la stabilité chimique des cristaux. Le solvant devrait être miscible à l'eau (diminution de l'eau résiduelle), former un azéotrope inférieur avec l'eau (distillation plus aisée des traces d'eau), avoir un point d'ébullition relativement bas (économie), être inerte chimiquement vis-à-vis du lactide (éviter l'ouverture du cycle), avoir une solubilité faible vis-à-vis du lactide (éviter les pertes de rendement), avoir une interaction avec l'eau supérieure à celle du lactide (éliminer l'eau liée au lactide). Le difficile choix du solvant résultera donc d'un compromis entre efficacité de l'extraction, rendement et rentabilité du procédé.

Les solvants utilisables sont des cétones, des éthers, des hydrocarbures aromatiques ou aliphatiques, des solvants à base de silicone et des solvants halogénés (acétone, 2-butanone, 2-pentanone, 2-hexanone, 2-heptanone, 2-octanone, anisole, éthyléther, isopropyléther, butyléther, méthylphényléther, méthylisobutylcétone, benzène, cumène, cymène, p-xylène, o-xylène, m-xylène, toluène, cyclohexane, hexane, heptane, octane, nonane, 1-pentène, 4-méthylanisole, 1,2-diméthoxybenzène, 1,3-diméthoxybenzène, 1,4-diméthoxybenzène, mésitylène, chlorobenzène, 1,2-dichlorobenzène, 1,3-dichlorobenzène, 1,4-dichlorobenzène, 2-chlorotoluène, 3-chlorotoluène, 4-chlorotoluène, éthanol, isopropanol).

### (c) le séchage

Cette étape consiste, au départ d'un gâteau humide obtenu en (b) et dont la teneur en eau est comprise entre 0 et 5 %, préférablement entre 0 et 2 % et plus préférablement encore entre 0 et 1 %, la teneur en un lactide (complexe inclus) comprise entre 75 et 98 %, préférablement entre 85 et 98 % et plus préférablement encore entre 90 et 98 %, la teneur en acide lactique et ses oligomères (LₙA avec n<5) comprise entre 0 et 5 %, préférablement entre 0 et 3 % et plus préférablement encore entre 0 et 1 %, le solde étant du méso-LD et l'autre diastéréoisomère du lactide, en une évaporation des teneurs résiduelles d'eau. Cette étape peut également permettre d'extraire totalement ou partiellement le solvant introduit lors du lavage.

De plus, le complexe généré par ce procédé devra être minutieusement géré : sa formation étant réversible, le complexe pourrait donc relarguer son eau sous certaines conditions.

Le gâteau humide issu de l'étape (b) devra donc être traité en considérant qu'il renferme une eau résiduelle libre, et une eau liée (sous forme de complexe).

La faible teneur en eau (libre +liée) du lactide humide, indispensable pour assurer la stabilité chimique temporaire du lactide, ne permet cependant pas une ultime purification par recristallisation en milieu fondu : cette teneur réduit le rendement par ouverture de cycle lors des refontes. Une approche privilégiée permet d'obtenir une teneur en eau résiduelle libre comprise entre 0 et 800 ppm, préférablement entre 0 et 600 ppm, plus préférablement entre 0 et 400 ppm. De même, la teneur en complexe devra être comprise entre 0 et 3%, préférablement entre 0 et 0,5% et plus préférablement entre 0 et 0,05%.

Dans le présent cadre, deux techniques de séchage pourront être envisagées pour traiter ces teneurs en eaux.

La première consiste à traiter le produit issu de l'étape (b) sous sa forme initiale (solide). Un sécheur offrant une capacité de volatilisation importante afin d'éliminer l'eau ou le solvant résiduel, effectuera le séchage dans des conditions douces et contrôlées pour éviter toute détérioration thermique du produit. L'opération de séchage est plus délicate car le gâteau humide contient, contrairement aux autres procédés d'extraction à l'eau, du méso-LD avec point de fusion entre 45 et 50°C. Il convient d'effectuer le séchage à moins de 50°C, sous vide ou sous flux gazeux. De plus, le complexe susceptible de relarguer de l'eau incite aussi à travailler à faible température afin d'éviter une dégradation du lactide. Tout procédé de séchage et toute technologie connue de l'homme de l'art, favorisant la vaporisation et l'extraction de l'eau ou d'un solvant d'un solide humide, seront envisagés dans cette invention : les mélangeurs sécheurs sous vide ou sous flux gazeux sec, le principe de zéodratation, les sécheurs à plateaux, etc.

La seconde technique préconise de liquéfier le gâteau humide de l'étape (b) et d'entraîner l'eau (libre + liée) par balayage ou barbotage d'un flux gazeux sec. Comme dans la première technique pour assurer la stabilité chimique du lactide, la température de séchage sera proche du minimum requis pour maintenir liquide le lactide humide. Tout procédé et technologie de séchage connue de l'homme de l'art, favorisant l'extraction de l'eau ou d'un solvant d'un liquide humide seront envisagés dans ce cadre : une colonne de lavage de gaz (striping), un sécheur couche mince, des tamis moléculaires, etc.

Par contre, si lors du lavage du gâteau par solvant, la teneur en eau résiduelle après extraction est compatible avec la recristallisation en milieu fondu décrite et si la quantité résiduelle de solvant reste compatible du point de vue chimique (pas d'ouverture du lactide par le solvant) et technique à l'étape ultime (pas de diminution de la teneur en solvant durant le procédé), cette étape de séchage peut être évitée.

Pour le lavage du gâteau, le solvant formera idéalement un azéotrope avec l'eau : il sera aisé et rentable d'extraire les dernières traces d'eau et de solvant afin de permettre une exploitation rentable de l'ultime étape de purification.

### (d) recristallisation en milieu fondu

Le lactide impur séché obtenu en (c), de composition semblable au produit issu de l'étape (b) à l'exception de la teneur plus faible en eau libre et liée, subira une ultime purification par recristallisation en milieu fondu (un ou plusieurs étages), afin d'obtenir un lactide d'une pureté chimique et stéréospécifique suffisante pour la synthèse du PLA par ouverture de cycle. Une pureté suffisante implique une teneur en un lactide comprise entre 99,0 et 99,9 % et préférablement encore entre 99,5 et 99,9 %, une teneur en méso-LD comprise entre 0 et 0,5 % et préférablement entre 0 et 0,2 %, une teneur en eau comprise entre 0 et 100 ppm et de préférence entre 0 et 50 ppm, ainsi qu'une acidité comprise entre 0 et 10 méq/kg et de préférence entre 0 et 1 méq/kg.

Le lactide impur séché obtenu en (c) est fondu et subit un refroidissement contrôlé pour initier la cristallisation. Les impuretés seront concentrées dans la phase liquide. Après la cristallisation, la phase liquide est éliminée par gravité, laissant des cristaux enrobés d'un film d'impuretés. Afin de l'éliminer, une refonte partielle est opérée. Le liquide ainsi obtenu entraîne le film et est évacué par gravité. L'opération est répétée jusqu'à atteindre la pureté requise. Cette succession d'étapes peut être statique ou dynamique.

La pureté souhaitée atteinte, le contenu du cristallisoir est fondu et récupéré.

Cette étape de purification ultime n'est quantitativement, économiquement et énergétiquement exploitable que par traitement préalable du brut de lactide suivant les 3 étapes ci-dessus. Le produit arrivant à la recristallisation en milieu fondu sera d'une pureté supérieure à 90 % et préférablement encore supérieure à 95 % pour une exploitation viable du procédé. Une pureté insuffisante accroît considérablement le nombre d'opérations et donc les investissements.

Une teneur en eau libre faible (<800 ppm et préférablement <400 ppm) permet d'éviter une détérioration chimique rapide du lactide, et une chute de productivité et de rendement. L'eau se concentre dans la phase liquide de la première étape et provoque une ouverture prématurée du lactide en raison des cycles de chauffe inhérents à la technologie. Le procédé étant basé sur le recyclage des différentes fractions, la perte engendrée influe alors directement sur le rendement final. La teneur en complexe dans le produit arrivant à la cristallisation en milieu fondu devra être drastiquement réduite : les conditions de cette étape pourraient provoquer un relargage de l'eau provenant du complexe.

Le choix judicieux des paramètres de recristallisation en milieu fondu permet une récupération du méso-LD grâce aux faibles pertes à l'étape (a). Ce produit sert à la synthèse de PLA à cinétique de dégradation contrôlée.

Durant la dernière étape de l'invention, la viscosité des impuretés du produit à purifier influence fortement le coefficient de transfert de masse au cours de la cristallisation et donc directement la forme des cristaux, la vitesse de cristallisation et le rendement. L'ajout, au produit de départ de l'étape (d), d'un solvant permet de diminuer la viscosité. Ce solvant peut être mélangé au produit séché de l'étape (c) lors d'une purification sans solvant ou être le résidu de solvant introduit lors de l'étape (b) du procédé. Cette teneur peut varier suivant qu'une étape de séchage (c) a été réalisée ou pas.

Ce solvant devra être présent dans des concentrations permettant de maintenir l'exploitation industrielle de notre procédé, à savoir comprises entre 0 et 30 %, préférablement entre 0 et 20 % et plus préférablement entre 0 et 10 %. L'ajout d'une quantité trop importante reviendrait à une recristallisation en solution, nécessitant des cristallisoirs de capacité plus importante, annulant le bénéfice de l'usage d'un solvant. Celui-ci devra être inerte vis-à-vis du lactide et aisément recyclé dans le procédé global de production de PLA : citons par exemple les esters d'acide lactique ou un solvant de l'étape (b).

D'autres détails et particularités de l'invention donnés ci-après de façon non limitative à titre d'exemple, décrivent des formes possibles de réalisation.

### Exemples

### Exemple 1

Un échantillon (feed) de brut de lactide (0,696 kg) contenant 83% de L-LD, 8 % de méso-LD, 1,6% de complexe L-LD hydraté (complexe) et une acidité résiduelle de 570 méq/kg est introduit dans un cristallisoir constitué par un tube vertical en inox de 1 m de long et de 30 mm de diamètre. La double enveloppe du tube est alimentée en fluide caloporteur par un groupe de chauffe thermostatisé pour le contrôle des phases de cristallisation, de suage ou de refonte. Ce brut est fondu à 105°C.

Ensuite, la cristallisation est initiée sur la paroi par à une diminution progressive de la température du fluide caloporteur présent dans la double enveloppe. Pour éviter des occlusions et des inclusions au sein des cristaux purs, cette descente en température sera de 2 à 5°C/h. Une partie du brut est cristallisé sur les parois, alors que la partie centrale renferme la phase liquide (drain) contenant la majorité des impuretés.

Une fois le fluide caloporteur amené à 60°C, la phase liquide est extraite par gravité.

Les cristaux sont encore recouverts d'un film d'impuretés que l'étape de suage doit éliminer : la surface du tube va être très progressivement chauffée (de 60 à 98°C) de façon à faire fondre la surface des cristaux de moindre pureté, car leur point de fusion est inférieur à celui du produit pur. Suivant la nature du brut, la fraction de suage récoltée par gravité représente de 5 à 25 % de la charge initiale.

Enfin, le cristallisoir est amené (à 4°C/min) à la fusion du produit (97-102°C) afin de liquéfier l'ensemble récolté par gravité (melt).

Un produit final devant répondre aux spécifications d'un lactide pour synthèse du PLA, subira un deuxième, voire un troisième étage de purification par la même procédure.

Le tableau I montre l'enrichissement des fractions intermédiaires en impuretés ainsi que l'accroissement du rendement massique des fractions récoltées en fonction des étages.

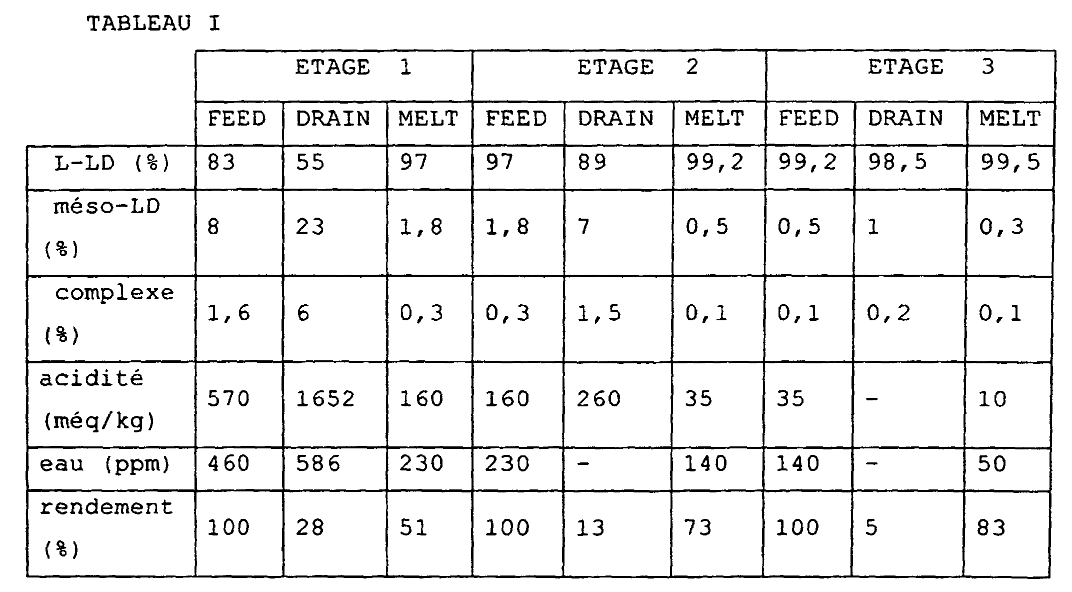

Les teneurs en L-LD, méso-LD et complexe sont déterminées par GC après estérification des composés carboxylés. Les acidités sont titrées au methoxyde de sodium dans un solvant anhydre avec de la phénolphtaléine comme indicateur. Les teneurs en eau ont été déterminées par Karl Fisher.

### Exemple 2

Un échantillon de brut de lactide contenant 77,2% de L-LD, 8,6 % de méso-LD, 1,2% de complexe et une acidité résiduelle de 1.840 méq/kg subira une pré-purification, avant une purification par recristallisation en milieu fondu comme à l'exemple 1.

Aux 2.583 kg de brut à 90°C sont ajoutés 25% en poids d'eau froide. Le mélange est rapidement amené à sa température de cristallisation et y restera 30 min afin de favoriser la nucléation des cristaux. Ensuite, la température est progressivement diminuée jusqu'à 25°C.

Le mélange est ensuite essoré à 1.500 tours/min et 1.553 kg de gros cristaux blancs sont récoltés. L'analyse de ce produit avant et après séchage, figure au tableau II.

**TABLEAU II**

| | Avant séchage | Après séchage |
|---|---|---|
| L-LD (%) | 94,3 | 85,8 |
| méso-LD (%) | 0,7 | 0,7 |
| complexe (%) | 3,5 | 11,8 |
| eau (ppm) | 5000 | 440 |

La teneur en eau trop importante pour une recristallisation directe en milieu fondu impose le barbotage d'un flux d'azote sec durant 1,5 h au sein du produit à 110°C. Ce traitement réduit la teneur en eau à 440 ppm mais augmente la concentration en complexe au détriment du L-LD.

Le produit séché issu de ce traitement subira deux ou trois étages de purification par recristallisation en milieu fondu, suivant l'exemple 1.

Le tableau III montre un accroissement de l'efficacité de la purification en milieu fondu. En effet, avec un feed de moindre pureté, deux étages suffisent pour atteindre la qualité requise. La présence du complexe influe fort négativement le rendement massique des fractions récoltées durant l'étape ultime de purification.

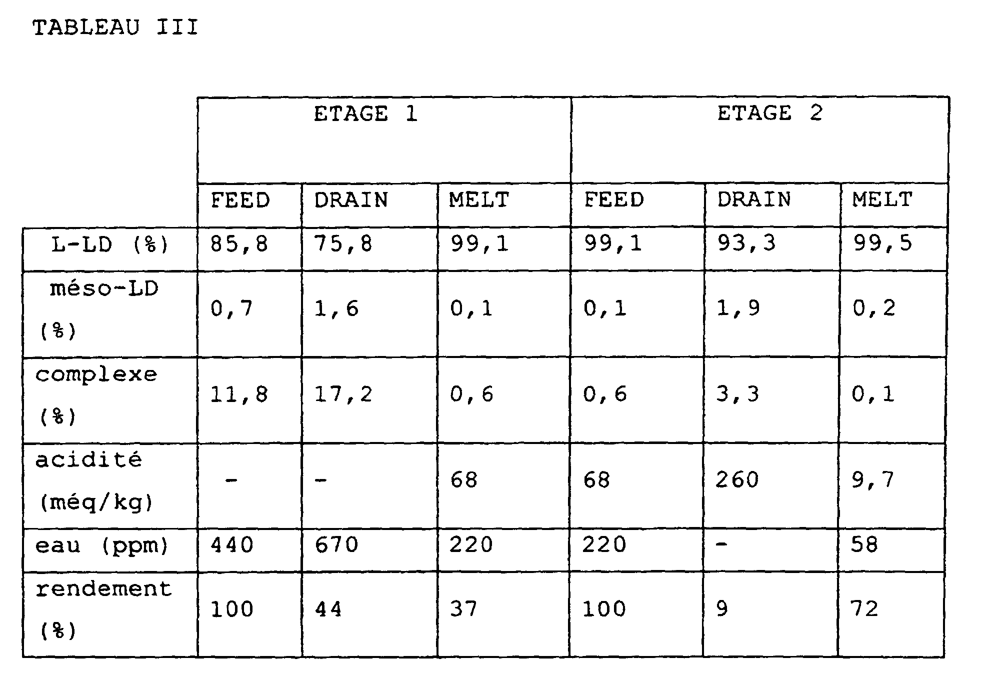

### Exemple 3

Un échantillon de brut de lactide contenant 84,9% de L-LD, 5,5 % de méso-LD, 3,3% de complexe et une acidité résiduelle de 830 méq/kg, subira un traitement selon l'exemple 2, mis à part les phases d'essorage et de séchage adaptées afin de minimiser la formation du complexe.

Aux 2.587 kg de brut à 90°C sont ajoutés 25% en poids d'eau froide. Le mélange est rapidement amené à sa température de cristallisation et y restera durant 30 min. Ensuite la température est diminuée jusqu'à 25°C.

Le mélange est ensuite essoré à 2.000 tours/min et 1.786 kg de gros cristaux blancs sont récoltés. Ces cristaux sont séchés sous vide à 45°C, pour extraire l'eau libre mais également l'eau liée sous forme de complexe. En effet, la disparition du complexe correspond à une augmentation de la teneur en L-LD. L'analyse du produit séché figure au tableau IV.

**TABLEAU IV**

| | Avant séchage | Après séchage |
|---|---|---|
| L-LD (%) | 90,8 | 97,6 |
| méso-LD (%) | 0,9 | 0,7 |
| complexe (%) | 5 | 1,1 |
| eau (ppm) | 3450 | 370 |

Le produit séché issu de ce traitement subira des recristallisations en milieu fondu suivant l'exemple 1.

Le tableau V montre par comparaison avec l'exemple 2, un accroissement du rendement massique des fractions récoltées dans ce procédé de purification lorsque la teneur en complexe dans le produit de départ est plus faible.

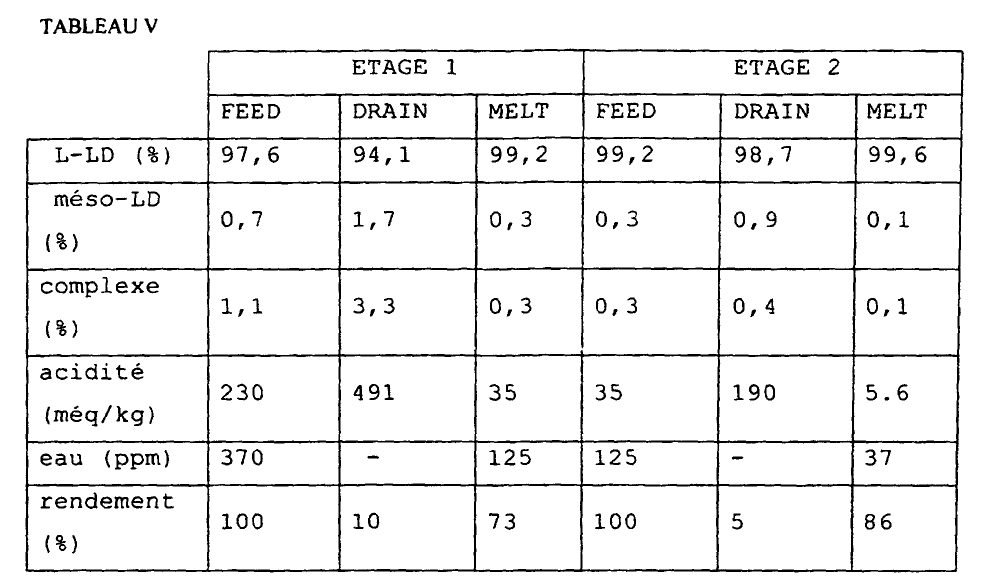

### Exemple 4

Cet exemple montre l'efficacité de la purification pour des mélanges pauvres en lactide, sous-produits du procédé de purification. Un échantillon de brut de lactide contenant 41,9% de L-LD, 14,3 % de méso-LD et 2,2% de complexe, subira un traitement suivant l'exemple 3.

Aux 1,082 kg de brut à 80°C sont ajoutés 25 % en poids d'eau froide. Le mélange est rapidement amené à sa température de cristallisation et y restera durant 30 min. Ensuite, la température est diminuée jusqu'à 25°C.

Le mélange est ensuite essoré et 0,400 kg de gros cristaux blancs sont récoltés et séchés. L'analyse du produit séché figure au tableau VI.

**TABLEAU VI**

| | Avant séchage | Après séchage |
|---|---|---|
| L-LD (%) | 91,1 | 93,2 |
| méso-LD (%) | 2,6 | 2,2 |
| complexe (%) | 2,4 | 0,8 |
| eau (ppm) | 4200 | 800 |

Le produit séché issu de ce traitement peut être traité par recristallisation en milieu fondu suivant l'exemple 1. Par rapport aux procédés conventionnels de production de PLA avec divers recyclages, notre technique offre l'avantage de recycler le lactide en tant que tel et non plus sous forme de lactate.

## Revendications

1. Procédé de purification de l'ester cyclique dimérique d'acide lactique au départ d'un brut de lactide comportant des impuretés, le procédé comprenant les étapes suivantes :
a) cristallisation extractive du brut de lactide en milieu aqueux avec contrôle de la géométrie des cristaux formés, en une phase solide comprenant l'un des diastéréoisomères du lactide et en une phase liquide comprenant l'acide lactique, le méso-lactide, l'autre diastéréoisomère du lactide, les oligomères ;
b) séparation de la suspension de cristaux obtenue en (a), en une phase liquide pauvre en lactide et chargée des impuretés et en un gâteau humide riche en cristaux de lactide;
c) séchage du gâteau humide obtenu en (b);
d) recristallisation en milieu fondu du lactide impur séché obtenu en (c) et récupération du lactide purifié.

2. Procédé suivant la revendication 1, **caractérisé en ce que** le brut de lactide comprend un mélange d'acide lactique et/ou d'ester d'acide lactique et leurs oligomères respectifs, d'eau et/ou d'alcool, ainsi que les différentes formes diastéréoisomères du lactide ou leurs mélanges.

3. Procédé suivant la revendication 2, **caractérisé en ce que** le brut de lactide est obtenu par mélange de fractions issues de procédés de synthèse ou de purification de lactide.

4. Procédé suivant l'une quelconque des revendications précédentes, **caractérisé en ce que** le brut de lactide présente une teneur en un diastéréoisomère du lactide comprise entre 30 et 90 % et préférablement comprise entre 40 et 85 %, une teneur en eau comprise entre 0 et 2 % et préférablement comprise entre 0 et 1 %, une teneur en acide lactique et oligomères d'acide lactique comprise entre 0 et 50 %, et une teneur en méso-lactide et en l'autre diastéréoisomère du lactide comprise entre 0 et 30 %.

5. Procédé suivant l'une quelconque des revendications précédentes, **caractérisé en ce que** la cristallisation extractive et contrôlée (a) comprend une première phase de germination progressive du lactide, et une seconde phase de croissance des cristaux avec rejet des impuretés en phase aqueuse.

6. Procédé suivant la revendication 5, **caractérisé en ce que** la phase de germination progressive est initiée par le maintien du mélange à une température légèrement inférieure à celle de cristallisation du lactide dans le mélange.

7. Procédé suivant la revendication 5, **caractérisé en ce que** la phase de croissance avec extraction des impuretés est assurée par une diminution contrôlée de la température du mélange, favorisant la croissance de cristaux de lactide.

8. Procédé suivant l'une quelconque des revendications précédentes, **caractérisé en ce que** la cristallisation extractive et contrôlée (a) est réalisée à une température comprise entre 100 et 0°C et préférablement comprise entre 80 et 10°C.

9. Procédé suivant l'une quelconque des revendications précédentes, **caractérisé en ce que** l'étape de cristallisation extractive et contrôlée (a) est effectuée sur un mélange dont la teneur en eau ajoutée par rapport au brut de lactide est comprise entre 0 et 40 %, de préférence comprise entre 0 et 30 %.

10. Procédé suivant l'une quelconque des revendications précédentes, **caractérisé en ce que** l'étape de cristallisation extractive et contrôlée (a) comprend l'utilisation d'un réacteur avec agitation, disposant d'une capacité de thermostatisation, et d'un système d'extraction également adapté aux produits pâteux, avec un temps de séjour compris entre 1 et 90 min, de préférence compris entre 1 et 60 min.

11. Procédé suivant l'une quelconque des revendications précédentes, **caractérisé en ce que** la composition de la suspension de cristaux obtenue après l'étape (a) comporte notamment une teneur en eau comprise entre 1 et 40 % et préférablement comprise entre 1 et 25 %, une teneur en un diastéréoisomère du lactide comprise entre 35 et 90 %, préférablement comprise entre 40 et 90 %, une teneur en acide lactique et oligomères d'acide lactique comprise entre 0 et 10 % et préférablement comprise entre 0 et 5 %, et une teneur en méso-lactide et en l'autre diastéréoisomère du lactide.

12. Procédé suivant la revendication 11, **caractérisé en ce que** la composition de la suspension de cristaux obtenue après l'étape (a) comporte le diastéréoisomère du lactide comprenant le lactide proprement dit et un complexe de lactide, qui consiste en une molécule de lactide liée de façon réversible, par pont d'hydrogène, à une molécule d'eau.

13. Procédé suivant l'une quelconque des revendications de procédé précédentes, **caractérisé en ce que** l'étape (b) de séparation est une séparation par centrifugation ou autre, qui permet d'atteindre une teneur résiduelle en eau libre dans le gâteau humide comprise entre 0 et 3 %, préférablement comprise entre 0 et 1 %.

14. Procédé suivant la revendication 13, **caractérisé en ce que** l'étape de séchage (c) est remplacée par un lavage centrifuge par solvant du gâteau humide riche en cristaux de lactide obtenu à l'étape (b), pour en extraire l'eau.

15. Procédé suivant l'une quelconque des revendications de procédé précédentes, **caractérisé en ce que** la composition du gâteau humide obtenu après l'étape (b) comporte notamment une teneur en eau libre comprise entre 0 et 5 % et préférablement comprise entre 0 et 2 %, une teneur totale en lactide comprise entre 75 et 98 %, préférablement comprise entre 85 et 98 %, une teneur en acide lactique et oligomères d'acide lactique comprise entre 0 et 5 %, préférablement comprise entre 0 et 3 %, et une teneur en méso-lactide et en l'autre diastéréoisomère du lactide.

16. Procédé suivant la revendication 15, **caractérisée en ce que** le lactide total dans le gâteau humide obtenu après l'étape (b) comprend le lactide diastéréoisomère souhaité et le lactide complexé.

17. Procédé suivant l'une quelconque des revendications de procédé précédentes, **caractérisé en ce que** l'étape (c) comprend un séchage qui atteint une teneur résiduelle en eau libre dans le lactide impur séché comprise entre 0 et 800 ppm, préférablement comprise entre 0 et 400 ppm, et une teneur résiduelle en eau liée sous forme de lactide complexe comprise entre 0 et 3 %, préférablement comprise entre 0 et 0,5 %.

18. Procédé suivant l'une quelconque des revendications de procédé précédentes, **caractérisé en ce que** le séchage (c) en phase solide du gâteau humide issu de (b) est réalisé sous vide ou sous flux gazeux sec, à une température inférieure à 50°C.

19. Procédé suivant l'une quelconque des revendications de procédé précédentes, **caractérisé en ce que** l'étape de séchage (c) en phase liquide comprend la liquéfaction préalable du gâteau humide issu de (b) et un entraînement de l'eau (libre et liée) par un barbotage et/ou balayage d'un flux gazeux sec dans la solution.

20. Procédé suivant la revendication 19, **caractérisé en ce que** la température de séchage (c) légèrement supérieure à la température de liquéfaction du lactide humide, est comprise entre 90 et 130°C et préférablement comprise entre 95 et 115°C.

21. Procédé suivant la revendication 18 ou 19, **caractérisée en ce que** le flux gazeux sec est un gaz inerte ou de l'air et **en ce que** ce flux gazeux est éventuellement préchauffé.

22. Procédé suivant l'une quelconque des revendications de procédé précédentes, **caractérisé en ce que** l'étape (d) comporte une ou plusieurs recristallisations en milieu fondu du lactide impur séché obtenu en (c).

23. Procédé suivant la revendication 22, **caractérisé en ce qu'**un réducteur de viscosité est mélangé au lactide impur séché issu de l'étape (c) afin d'augmenter la vitesse de cristallisation, le coefficient de transfert de masse et l'efficacité de la recristallisation en milieu fondu.

24. Procédé suivant la revendication 23, **caractérisé en ce que** le réducteur de viscosité est un solvant introduit à l'issue de l'étape de séchage (c), et choisi parmi les cétones, les éthers, les solvants aromatiques ou aliphatiques, les solvants à base de silicone, les solvants halogénés, les alcools et les esters d'acide lactique.

25. Procédé suivant la revendication 23, **caractérisé en ce que** le réducteur de viscosité est le solvant résiduel introduit lors de l'étape de séparation centrifuge (b) du procédé, et choisi parmi les cétones, les éthers, les solvants aromatiques ou aliphatiques, les solvants à base de silicone, les solvants halogénés, les alcools et les esters d'acide lactique.

26. Procédé suivant l'une quelconque des revendications 23 à 25, **caractérisé en ce que** la teneur en réducteur de viscosité est comprise entre 0 et 30 %, préférablement comprise entre 0 et 20 %.

27. Procédé suivant l'une quelconque des revendications de procédé précédentes, **caractérisé en ce que** la teneur en lactide à l'issue de l'étape (d) est comprise entre 99,0 et 99,9 %, préférablement comprise entre 99,5 et 99,9 %, la teneur en méso-lactide est comprise entre 0 et 0,5 %, préférablement comprise entre 0 et 0,2 %, la teneur en eau est comprise entre 0 et 100 ppm, préférablement comprise entre 0 et 50 ppm, la teneur acide lactique et oligomères est comprise entre 0 et 10 méq/kg, préférablement comprise entre 0 et 1 méq/kg.

## Patentansprüche

1. Verfahren zur Reinigung des dimeren cyclischen Esters von Milchsäure ausgehend von einem Verunreinigungen enthaltenden Lactidrohprodukt mit den folgenden Schritten:
a) extraktive Kristallisation des Lactidrohprodukts aus einem wäßrigen Medium mit Steuerung der Geometrie der gebildeten Kristalle unter Bildung einer festen Phase, die eines der Diastereomere des Lactids enthält, und eine flüssige Phase, die Milchsäure, meso-Lactid, das andere Lactid-Diastereomer und Oligomere enthält;
b) Trennung der unter (a) erhaltenen Kristallsuspension in eine lactidarme und mit Verunreinigungen beladene flüssige Phase und einen lactidkristallreichen feuchten Kuchen;
c) Trocknen des unter (b) erhaltenen feuchten Kuchens;
d) Umkristallisation des unter (c) erhaltenen getrockneten unreinen Lactids aus der Schmelze und Rückgewinnung des gereinigten Lactids.

2. Verfahren nach Anspruch 1, **dadurch gekennzeichnet, daß** das Lactidrohprodukt ein Gemisch aus Milchsäure und/oder Milchsäureester und deren jeweiligen Oligomeren, Wasser und/oder Alkohol sowie verschiedene diastereomere Formen des Lactids oder Gemische davon enthält.

3. Verfahren nach Anspruch 2, **dadurch gekennzeichnet, daß** das Lactidrohprodukt durch Mischen von Fraktionen aus Verfahren zur Synthese oder Reinigung von Lactid erhalten wird.

4. Verfahren nach einem der vorhergehenden Ansprüche, **dadurch gekennzeichnet, daß** das Lactidrohprodukt einen Gehalt an einem Lactid-Diastereomer zwischen 30 und 90% und vorzugsweise zwischen 40 und 85%, einen Wassergehalt zwischen 0 und 2% und vorzugsweise zwischen 0 und 1%, einen Gehalt an Milchsäure und Milchsäureoligomeren zwischen 0 und 50% und einen Gehalt an meso-Lactid und dem anderen Lactid-Diastereomer zwischen 0 und 30% aufweist.

5. Verfahren nach einem der vorhergehenden Ansprüche, **dadurch gekennzeichnet, daß** die gesteuerte extraktive Kristallisation (a) eine erste Phase der progressiven Lactidkeimbildung und eine zweite Phase des Kristallwachstums mit Abgang von Verunreinigungen in die wäßrige Phase umfaßt.

6. Verfahren nach Anspruch 5, **dadurch gekennzeichnet, daß** man die Phase der progressiven Keimbildung initiiert, indem man das Gemisch bei einer etwas unter der Kristallisationstemperatur des Lactids im Gemisch liegenden Temperatur hält.

7. Verfahren nach Anspruch 5, **dadurch gekennzeichnet, daß** man die Phase des Wachstums mit Extraktion von Verunreinigungen gewährleistet, indem man die Temperatur des Gemischs kontrolliert verringert, wodurch das Lactidkristallwachstum gefördert wird.

8. Verfahren nach einem der vorhergehenden Ansprüche, **dadurch gekennzeichnet, daß** man die gesteuerte extraktive Kristallisation (a) bei einer Temperatur zwischen 100 und 0°C und vorzugsweise zwischen 80 und 10°C durchführt.

9. Verfahren nach einem der vorhergehenden Ansprüche, **dadurch gekennzeichnet, daß** man den Schritt der gesteuerten extraktiven Kristallisation (a) an einem Gemisch mit einem Gehalt an zugesetztem Wasser, bezogen auf das Lactidrohprodukt, zwischen 0 und 40% und vorzugsweise 0 bis 30% durchführt.

10. Verfahren nach einem der vorhergehenden Ansprüche, **dadurch gekennzeichnet, daß** man im Schritt der gesteuerten extraktiven Kristallisation (a) einen thermostatisierbaren Rührreaktor und ein auch für pastöse Produkte ausgelegtes Extraktionssystem verwendet, wobei die Verweilzeit zwischen 1 und 90 min und vorzugsweise zwischen 1 und 60 min liegt.

11. Verfahren nach einem der vorhergehenden Ansprüche, **dadurch gekennzeichnet, daß** die Zusammensetzung der nach Schritt (a) erhaltenen Kristallsuspension insbesondere einen Wassergehalt zwischen 1 und 40% und vorzugsweise zwischen 1 und 25%, einen Gehalt an einem Lactid-Diastereomer zwischen 35 und 90% und vorzugsweise zwischen 40 und 90%, einen Gehalt an Milchsäure und Milchsäureoligomeren zwischen 0 und 10% und vorzugsweise zwischen 0 und 5% und einen Gehalt an meso-Lactid und dem anderen Lactid-Diastereomer aufweist.

12. Verfahren nach Anspruch 11, **dadurch gekennzeichnet, daß** die Zusammensetzung der nach Schritt (a) erhaltenen Kristallsuspension das Lactid-Diasteromer, umfassend das eigentliche Lactid-Diastereomer und einen Lactid-Komplex, der aus einem Lactidmolekül, das über Wasserstoffbrücken reversibel an ein Wassermolekül gebunden ist, besteht, enthält.

13. Verfahren nach einem der vorhergehenden Ansprüche, **dadurch gekennzeichnet, daß** es sich bei dem Trennschritt (b) um eine Trennung durch Zentrifugation oder eine andere Trennung handelt, mit der man einen Restgehalt an freiem Wasser in dem feuchten Kuchen zwischen 0 und 3% und vorzugsweise zwischen 0 und 1% erreichen kann.

14. Verfahren nach Anspruch 13, **dadurch gekennzeichnet, daß** man den Trocknungsschritt (c) durch eine Zentrifugenwäsche des aus Schritt (b) erhaltenen lactidkristallreichen feuchten Kuchens mit Lösungsmittel zwecks Extraktion des Wassers ersetzt.

15. Verfahren nach einem der vorhergehenden Ansprüche, **dadurch gekennzeichnet, daß** die Zusammensetzung des nach Schritt (b) erhaltenen feuchten Kuchens einen Gehalt an freiem Wasser zwischen 0 und 5% und vorzugsweise zwischen 0 und 2%, einen Lactidgesamtgehalt zwischen 75 und 98% und vorzugsweise zwischen 85 und 98%, einen Gehalt an Milchsäure und Milchsäureoligomeren zwischen 0 und 5% und vorzugsweise zwischen 0 und 3% und einen Gehalt an meso-Lactid und dem anderen Lactid-Diastereomer aufweist.

16. Verfahren nach Anspruch 15, **dadurch gekennzeichnet, daß** das Gesamtlactid im nach Schritt (b) erhaltenen feuchten Kuchen das gewünschte Lactid-Diastereomer und das komplexierte Lactid umfasst.

17. Verfahren nach einem der vorhergehenden Ansprüche, **dadurch gekennzeichnet, daß** Schritt (c) eine Trocknung umfaßt, bei der man einen Restgehalt an freiem Wasser in dem getrockneten unreinen Lactid zwischen 0 und 800 ppm und vorzugsweise zwischen 0 und 400 ppm und einen Restgehalt an Wasser in Form von Lactid-Komplex zwischen 0 und 3% und vorzugsweise zwischen 0 und 0,5% erzielt.

18. Verfahren nach einem der vorhergehenden Ansprüche, **dadurch gekennzeichnet, daß** man die Festphasentrocknung (c) des feuchten Kuchens aus (b) unter Vakuum oder unter einem Trockengasstrom bei einer Temperatur unter 50°C durchführt.

19. Verfahren nach einem der vorhergehenden Ansprüche, **dadurch gekennzeichnet, daß** der Flüssigphasentrocknungsschritt (c) die vorherige Verflüssigung des feuchten Kuchens aus (b) und ein Austreiben von Wasser (frei und gebunden) durch Einblasen eines Trockengasstroms in die Lösung und/oder Spülen der Lösung mit einem Trockengasstrom umfaßt.

20. Verfahren nach Anspruch 19, **dadurch gekennzeichnet, daß** die etwas über der Verflüssigungstemperatur des feuchten Lactids liegende Temperatur der Trocknung (c) zwischen 90 und 130°C und vorzugsweise zwischen 95 und 115°C liegt.

21. Verfahren nach Anspruch 18 oder 19, **dadurch gekennzeichnet, daß** es sich bei dem Trockengasstrom um ein Inertgas oder Luft handelt und dieser Gasstrom gegebenenfalls vorerhitzt wird.

22. Verfahren nach einem der vorhergehenden Ansprüche, **dadurch gekennzeichnet, daß** Schritt (d) eine oder mehrere Umkristallisationen aus der Schmelze des unter (c) erhaltenen getrockneten unreinen Lactids umfaßt.

23. Verfahren nach Anspruch 22, **dadurch gekennzeichnet, daß** man dem unter (c) erhaltenen getrockneten unreinen Lactid ein die Viskosität verringerndes Mittel beimischt, um die Kristallisationsgeschwindigkeit, den Stoffübergangskoeffizienten und die Effizienz der Umkristallisation aus der Schmelze zu erhöhen.

24. Verfahren nach Anspruch 23, **dadurch gekennzeichnet, daß** das die Viskosität verringernde Mittel ein am Ende des Trocknungsschritts (c) eingetragenes Lösungsmittel ist und unter Ketonen, Ethern, aromatischen oder aliphatischen Lösungsmitteln, Lösungsmitteln auf Silikonbasis, halogenierten Lösungsmitteln, Alkoholen und Milchsäureestern ausgewählt wird.

25. Verfahren nach Anspruch 23, **dadurch gekennzeichnet, daß** das die Viskosität verringernde Mittel das im Zentrifugentrennschritt (b) eingetragene Restlösungsmittel ist und unter Ketonen, Ethern, aromatischen oder aliphatischen Lösungsmitteln, Lösungsmitteln auf Silikonbasis, halogenierten Lösungsmitteln, Alkoholen und Milchsäureestern ausgewählt wird.

26. Verfahren nach einem der Ansprüche 23 bis 25, **dadurch gekennzeichnet, daß** der Gehalt an die Viskosität verringerndem Mittel zwischen 0 und 30% und vorzugsweise zwischen 0 und 20% liegt.

27. Verfahren nach einem der vorhergehenden Ansprüche, **dadurch gekennzeichnet, daß** der Lactidgehalt am Ende von Schritt (d) zwischen 99,0 und 99,9% und vorzugsweise zwischen 99,5 und 99,9% liegt, der Gehalt an meso-Lactid zwischen 0 und 0,5% und vorzugsweise zwischen 0 und 0,2% liegt, der Wassergehalt zwischen 0 und 100 ppm und vorzugsweise zwischen 0 und 50 ppm liegt und der Gehalt an Milchsäure und Oligomeren zwischen 0 und 10 mÄq./kg und vorzugsweise zwischen 0 und 1 mÄq./kg liegt.

## Claims

1. A process for purifying the dimeric cyclic ester of lactic acid starting from a crude lactide comprising impurities, the process comprising the following steps:
a) extractive crystallization of the crude lactide in an aqueous medium, controlling the geometry of the crystals formed, producing a solid phase that comprises one of the lactide diastereoisomers and a liquid phase that comprises the lactic acid, the meso-lactide, the other lactide diastereoisomer and the oligomers;
b) separation of the crystal suspension obtained in (a) into a low-lactide liquid phase loaded with impurities and a lactide-crystal-rich wet cake;
c) drying of the wet cake obtained in (b);
d) recrystallization in a melt medium of the dried impure lactide obtained in (c), and recovery of the purified lactide.

2. The process of claim 1, **characterized in that** the crude lactide comprises a mixture of lactic acid and/or lactic ester and their respective oligomers, water and/or alcohol, and the various diastereoisomeric forms of the lactide or mixtures thereof.

3. The process of claim 2, **characterized in that** the crude lactide is obtained by mixing fractions originating from lactide purification or synthesis processes.

4. The process of any one of the preceding claims, **characterized in that** the crude lactide has a content of one diastereoisomer of the lactide of between 30 and 90% and preferably between 40 and 85%, a water content of between 0 and 2% and preferably between 0 and 1%, a content of lactic acid and oligomers of lactic acid of between 0 and 50%, and a content of meso-lactide and the other diastereoisomer of the lactide of between 0 and 30%.

5. The process of any one of the preceding claims, **characterized in that** the controlled, extractive crystallization (a) comprises a first phase of progressive seeding of the lactide and a second phase of crystal growth with expulsion of the impurities into the aqueous phase.

6. The process of claim 5, **characterized in that** the progressive seeding phase is initiated by maintaining the mixture at a temperature which is slightly lower than the crystallization temperature of the lactide in the mixture.

7. The process of claim 5, **characterized in that** the growth phase with extraction of the impurities is assured by a controlled reduction in the temperature of the mixture, promoting the growth of lactide crystals.

8. The process of any one of the preceding claims, **characterized in that** the controlled, extractive crystallization (a) is carried out at a temperature of between 100 and 0°C and preferably between 80 and 10°C.

9. The process of any one of the preceding claims, **characterized in that** the controlled, extractive crystallization step (a) is carried out on a mixture in which the amount of added water relative to the crude lactide is between 0 and 40%, preferably between 0 and 30%.

10. The process of any one of the preceding claims, **characterized in that** the controlled, extractive crystallization step (a) comprises the use of a reactor with stirring, having a thermostating capacity, and an extraction system which is also suitable for pastelike products, with a residence time of between 1 and 90 min, preferably between 1 and 60 min.

11. The process of any of the preceeding claims, **characterized in that** the composition of the crystal suspension obtained after step (a), comprises in particular a water content of between 1 and 40% and preferably between 1 and 25%, a content of one diastereoisomer of the lactide of between 35 and 90%, preferably between 40 and 90%, a content of lactic acid and oligomers of lactic acid of between 0 and 10% and preferably between 0 and 5%, and a content of meso-lactide and the other diastereoisomer of the lactide.

12. The process of claim 11, **characterized in that** the composition of the crystal suspension obtained after step (a), comprises the diastereoisomer of the lactide which includes the lactide per se and a lactide complex consisting in one molecule of lactide bound reversibly by hydrogen bonding to one molecule of water.

13. The process of any one of the preceding process claims, **characterized in that** the separation step (b) is a centrifugation separation or other separation which makes it possible to obtain a residual free water content in the wet cake of between 0 and 3%, preferably between 0 and 1%.

14. The process of claim 13, **characterized in that** the drying step (c) is replaced by centrifugal solvent washing of the lactide-crystal-rich wet cake obtained in step (b) in order to extract the water from it.

15. The process of any of the preceding claims, **characterized in that** the composition of the wet cake obtained after step (b), comprises in particular a free water content of between 0 and 5% and preferably between 0 and 2%, a total lactide content of between 75 and 98%, preferably between 85 and 98%, a content of lactic acid and oligomers of lactic acid of between 0 and 5%, preferably between 0 and 3%, and a content of meso-lactide and the other diastereoisomer of the lactide.

16. The process of claim 15, **characterized in that** the total lactide in the wet cake obtained after step (b), comprises the desired lactide diastereoisomer and the complexed lactide.

17. The process of any one of the preceding process claims, **characterized in that** step (c) comprises a drying procedure which attains a residual free water content in the dried impure lactide of between 0 and 800 ppm, preferably between 0 and 400 ppm, and a residual content of water bound in the form of lactide complex of between 0 and 3%, preferably between 0 and 0.5%.

18. The process of any one of the preceding process claims, **characterized in that** the drying (c) in solid phase of the wet cake obtained from (b) is carried out under vacuum or under a dry gas stream at a temperature of less than 50°C.

19. The process of any one of the preceding process claims, **characterized in that** the step of drying (c) in liquid phase comprises the prior liquefaction of the wet cake obtained from (b) and then entrainment of the water (free and bound) by sparging and/or purging of a dry gas stream into the solution.

20. The process of claim 19, **characterized in that** the temperature of drying (c), which is slightly greater than the temperature of liquefaction of the wet lactide, is between 90 and 130°C and preferably between 95 and 115°C.

21. The process of claim 18 or 19, **characterized in that** the dry gas stream is an inert gas or air and **in that** this gas stream is preheated where appropriate.

22. The process of any one of the preceding process claims, **characterized in that** step (d) comprises one or more recrystallizations in a melt medium of the dried impure lactide obtained in (c).

23. The process of claim 22, **characterized in that** a viscosity reducer is mixed with the dried impure lactide obtained from step (c) in order to increase the rate of crystallization, the mass transfer coefficient, and the efficiency of the recrystallization in a melt medium.

24. The process of claim 23, **characterized in that** the viscosity reducer is a solvent which is introduced at the end of the drying step (c) and is selected from ketones, ethers, aromatic or aliphatic solvents, silicone-based solvents, halogenated solvents, alcohols, and esters of lactic acid.

25. The process of claim 23, **characterized in that** the viscosity reducer is a residual solvent introduced during the step of centrifugal separation (b) of the process and is selected from ketones, ethers, aromatic or aliphatic solvents, silicone-based solvents, halogenated solvents, alcohols, and esters of lactic acid.

26. The process of any one of claims 23 to 25, **characterized in that** the viscosity reducer content is between 0 and 30%, preferably between 0 and 20%.

27. The process of any one of the preceding process claims, **characterized in that** the lactide content at the end of step (d) is between 99.0 and 99.9%, preferably between 99.5 and 99.9%, the meso-lactide content is between 0 and 0.5%, preferably between 0 and 0.2%, the water content is between 0 and 100 ppm, preferably between 0 and 50 ppm, the amount of lactic acid and oligomers is between 0 and 10 meq/kg, preferably between 0 and 1 meq/kg.
